# EUROPÄISCHE PATENTANMELDUNG

(11) **EP 1 300 795 A2**
(43) Veröffentlichungstag der Anmeldung: **09.04.2003**
(21) Anmeldenummer: 02021044.9
(22) Anmeldetag: 20.09.2002
(51) Int. Cl.: G06F 19/00

(54) **Verfahren und System zum Fernüberwachen einer Wirkung eines Medikaments**

(30) Priorität: 04.10.2001 DE 10148838
(71) Anmelder: SIEMENS AKTIENGESELLSCHAFT, 80333 München (DE)
(72) Erfinder: Schmidt, Volker, 91054 Erlangen (DE)

(57) **Zusammenfassung**

Die Erfindung betrifft ein Verfahren zum Fernüberwachen einer Wirkung eines Medikamentes (4), das eine Person (1) einnimmt. Die Erfindung betrifft ferner ein System zum Fernüberwachen einer Wirkung eines Medikamentes (4), das eine Person (1) einnimmt, aufweisend eine Datenbank (11a), in der eine der Person (1) und dem Medikament (4) zugeordnete elektronische Überwachungsakte (45) und für eine Beschreibung einer erwarteten Wirkung des Medikamentes (4) geeignete Daten gespeichert sind, Mittel (11b) zum Empfangen von für die Beschreibung der tatsächlichen Wirkung des Medikamentes (4) geeigneten Daten und Mittel zum Vergleichen der für die Beschreibung der tatsächlichen Wirkung des Medikamentes (4) geeigneten Daten mit den für die Beschreibung der erwarteten Wirkung des Medikamentes (4) geeigneten Daten. Die Erfindung betrifft außerdem ein Rechnerprogramm, das das erfindungsgemäße Verfahren bzw. das erfindungsgemäße System implementiert und eine Datenverarbeitungsanlage (11), auf der das Rechnerprogramm gespeichert ist.

## Beschreibung

Die Erfindung betrifft ein Verfahren und ein System zum Fernüberwachen einer Wirkung eines Medikamentes, das eine Person einnimmt.

In der Regel verschreibt ein Arzt einem Patienten ein Medikament, damit sich dessen Gesundheitszustand verbessert oder zumindest nicht verschlechtert. Der Arzt kann zwar die Wirkung des Medikamentes überprüfen, wenn der Patient während oder nach einer Einnahme des Medikamentes den Arzt aufsucht. Eine systematische Überwachung der Wirkung des verschriebenen Medikamentes unterbleibt jedoch häufig.

Die Aufgabe der Erfindung ist daher, ein Verfahren anzugeben, das eine Voraussetzung für eine leichte und systematische Überwachung der Wirkung eines Medikamentes erlaubt. Eine weitere Aufgabe der Erfindung ist es, ein System, das eine leichte und systematische Überwachung der Wirkung des Medikamentes erlaubt, anzugeben.

Die erste Aufgabe der Erfindung wird gelöst durch ein Verfahren zum Fernüberwachen einer Wirkung eines Medikamentes, aufweisend folgende Verfahrensschritte:
a) Anlegen einer elektronischen Überwachungsakte, die einer das Medikament einnehmenden Person zugeordnet ist,
b) Empfangen von für eine Beschreibung der tatsächlichen Wirkung des Medikamentes geeigneten Daten,
c) Speichern der die Beschreibung der tatsächlichen Wirkung des Medikamentes geeigneten Daten in der elektronischen Überwachungsakte und
d) Vergleichen der die Beschreibung der tatsächlichen Wirkung des Medikamentes geeigneten Daten mit für eine Beschreibung einer erwarteten Wirkung des Medikamentes geeigneten Daten.

Erfindungsgemäß wird also zunächst die elektronische Überwachungsakte, die der das Medikament einnehmenden Person zugeordnet ist und in der die zur Beschreibung der tatsächlichen Wirkung des Medikamentes geeigneten Daten gespeichert werden, beispielsweise von dem die Person behandelnden Arzt angelegt. Das Anlegen der elektronischen Überwachungsakte und somit die Fernüberwachung der Wirkung des Medikamentes kann aber auch als Dienstleistung z.B. von dem Hersteller des Medikamentes, der Krankenkasse oder des Krankenversicherers der Person oder einem unabhängigen Dienstleister der Person als Dienstleistung angeboten werden, wobei für eine Wahrnehmung des Angebots notwendige Informationen, wie z.B. die Anschrift der anbietenden Institution, auf einem Beipackzettel des Medikamentes vermerkt sein können.

Mit dem Anlegen der Überwachungsakte werden neben der eventuell anonymisierten Identität der das Medikament einnehmenden Person auch Überwachungsparameter für die Fernüberwachung festgelegt. Diese Überwachungsparameter entsprechen den die erwartete Wirkung des Medikamentes beschreibenden Daten, mit denen die die tatsächliche Wirkung des Medikaments beschreibenden Daten erfindungsgemäß verglichen werden. Da die Person das Medikament in der Regel einnimmt, damit sich ihr Gesundheitszustand verbessert oder zumindest nicht verschlechtert, die Person das Medikament also für einen bestimmten Zweck einnimmt, ist folglich auch die gewünschte, also die erwartete Wirkung des Medikamentes bekannt. Die der erwarteten Wirkung des Medikamentes geeigneten Daten können z.B., wie es nach einer Variante der Erfindung vorgesehen ist, einer Diagnose, aufgrund deren die Person das Medikament einnimmt, einer Dosis des Medikamentes und/oder einer zu erwarteten Nebenwirkung zugeordnet sein. Eine erwartete Wirkung des Medikamentes kann aber auch, wie es gemäß einer weiteren Ausführungsform der Erfindung vorgesehen ist, ein erwarteter medizinischer Zustand der das Medikament einnehmenden Person sein. Damit eine wirkungsvolle Fernüberwachung realisierbar ist, können zusätzlich mit dem Anlegen der Überwachungsakte Zeitpunkte oder Zeitintervalle definiert werden, an denen die zur tatsächlichen Beschreibung der Wirkung des Medikamentes geeigneten Daten empfangen werden sollen.

Im Falle von Multimorbität und/oder einer Einnahme von mehreren Medikamenten kann auch ein Expertensystem verwendet werden, das aus den Diagnosen, Medikamenten und Dosierungen der einzelnen Medikamente für die Beschreibung der erwarteten Wirkungen der Medikamente geeignete Daten ermittelt.

Während der Zeitdauer der Fernüberwachung werden die zur Beschreibung der tatsächlichen Wirkung des Medikamentes geeigneten Daten empfangen und in der elektronischen Überwachungsakte gespeichert. Die tatsächliche Wirkung des Medikamentes lässt sich beispielsweise gemäß einer Variante der Erfindung durch einen tatsächlichen medizinischen Zustand der das Medikament einnehmenden Person beschreiben. Die Beschreibung des tatsächlichen medizinischen Zustands lässt sich z.B. nach einer Ausführungsform der Erfindung aufgrund des Blutdrucks, der Herzfrequenz, eines Brechreizes, eines Stuhlgangs, von Blutwerten und/oder einem allgemeinen Wohlbefinden der das Medikament einnehmenden Person ermitteln.

Der tatsächliche medizinische Zustand der das Medikament einnehmenden Person wird insbesondere gemäß einer Variante der Erfindung mit wenigstens einem Messgerät ermittelt. Das Messgerät kann beispielsweise der das Medikament einnehmenden Person während der Zeitdauer der Fernüberwachung von der die elektronische Überwachungsakte anlegenden Institution zur Verfügung gestellt werden.

Gemäß einer Variante der Erfindung ermittelt die das Medikament einnehmende Person selber oder eine weitere, insbesondere im Gesundheitswesen tätige Person den tatsächlichen medizinischen Zustand der das Medikament einnehmenden Person.

Die zur Beschreibung der tatsächlichen Wirkung des Medikamentes geeigneten Daten werden vorteilhaft gemäß einer Ausgestaltung der Erfindung über eine Datenübertragungseinrichtung an eine die elektronische Überwachungsakte umfassende Datenverarbeitungsanlage übermittelt. Eine Datenübertragungseinrichtung ist beispielsweise ein Telefon, ein Faxgerät oder das Internet. Somit kann die Person oder die weitere, insbesondere im Gesundheitswesen tätige Person die tatsächliche Wirkung des Medikaments z.B. der Institution, die die elektronische Überwachungsakte anlegte und die Fernüberwachung durchführt, telefonisch durchgeben. Auch ist es denkbar, dass die besagte Institution der das Medikament einnehmenden Person eine ihr zugeordnete Webseite auf der Datenverarbeitungsanlage bereitstellt. Die Webseite umfasst z.B. einen Fragebogen, mit dessen Hilfe die tatsächliche Wirkung des Medikamentes ermittelt werden kann. Die das Medikament einnehmende Person oder die weitere, insbesondere im Gesundheitswesen tätige Person kann diese Webseite mit einem an das Internet angeschlossenen PC herunterladen, ausfüllen und die ausgefüllte Webseite an die Datenverarbeitungsanlage übermitteln. Somit ist eine einfache und zuverlässige Übermittlung der die tatsächliche Wirkung des Medikamentes beschreibenden Daten gegeben.

Nach einer weiteren Ausführungsform der Erfindung wird ein Alarmsignal ausgelöst, wenn die tatsächliche Wirkung des Medikamentes von der erwarteten Wirkung um mehr als ein vorgegebenes Maß abweicht. Tritt beispielsweise die gewünschte Wirkung des Medikamentes nicht ein, wird das Alarmsignal zuverlässig ausgelöst. Das Alarmsignal kann z.B. dem die Person behandelnden Arzt, einem Krankenhaus oder auch Angehörigen der das Medikament einnehmenden Person übermittelt werden. Somit kann der das Medikament einnehmenden Person insbesondere schnell geholfen werden, wenn aufgrund der Einnahme des Medikamentes der tatsächliche medizinische Zustand der Person bedenklich wird.

Nach einer Ausführungsform der Erfindung wird das Medikament während einer Medikamentenwirksamkeitsstudie insbesondere während der Phase III und IV eingenommen. Medikamentenwirksamkeitsstudien werden in vier Phasen unterteilt. Phase I umfasst dabei im Wesentlichen Tierversuche und einzelne Menschenversuche, Phase II umfasst kleine Menschengruppen, die unter strenger klinischer Beobachtung das Medikament während der Medikamentenverträglichkeitsstudie einnehmen. Phase III ist dagegen eine Doppelblindstudie, welche für die Zulassung beispielsweise für die FDA (US Food and Drug Administration) oder für das Bundesamt für Arzneimittel nötig ist. Bei der Phase IV handelt es sich dabei um eine Studie, in der untersucht werden soll, ob ein für eine bestimmte medizinische Indikation bereits zugelassenes Medikament auch für andere medizinische Indikationen zugelassen werden kann.

Die Medikamentenwirksamkeitsstudie kann dann insbesondere in vorteilhafter Weise durchgeführt werden, wenn nach einer weiteren Ausführungsform der Erfindung eine Webseite vorgesehen ist, mittels derer sich insbesondere das Medikament einnehmende Personen während der Medikamentenverträglichkeitsstudie über auftretende Nebenwirkungen informieren können.

Nach einer weiteren Variante der Erfindung ist ferner eine Webseite vorgesehen, auf der das Medikament einnehmende Personen während der Medikamentenverträglichkeitsstudie von aufgrund der Einnahme des Medikamentes hervorgerufenen Nebenwirkungen berichten können.

Die zweite Aufgabe der Erfindung wird gelöst durch ein System zum Fernüberwachen einer Wirkung eines Medikamentes, das eine Person einnimmt, aufweisend eine Datenbank, in der eine der Person und dem Medikament zugeordnete elektronische Überwachungsakte und für eine Beschreibung einer erwarteten Wirkung des Medikamentes geeignete Daten gespeichert sind, Mittel zum Empfangen von für die Beschreibung der tatsächlichen Wirkung des Medikamentes geeigneten Daten und Mittel zum Vergleichen der für die Beschreibung der tatsächlichen Wirkung des Medikamentes geeigneten Daten mit den für die Beschreibung der erwarteten Wirkung des Medikamentes geeigneten Daten. Vorteilhafte Ausgestaltungen des erfindungsgemäßen Systems ergeben sich aus den Unteransprüchen.

Das erfindungsgemäße System ist also derart ausgeführt, dass mit ihm das erfindungsgemäße Verfahren ausgeführt werden kann.

Die Aufgabe der Erfindung wird auch gelöst durch ein Rechnerprogramm, das das erfindungsgemäße Verfahren implementiert und ein Rechnerprogramm, das das erfindungsgemäße System implementiert. Die Aufgabe der Erfindung wird ebenfalls gelöst durch eine Datenverarbeitungsanlage, auf der eine der erfindungsgemäßen Rechnerprogramme implementiert ist.

Ein Grundgedanke der Erfindung ist, die Verschreibung und Einnahme eines Medikamentes mit einer Fernüberwachung der Wirkung des Medikamentes zu koppeln. Die Fernüberwachung kann als Dienstleistung angeboten werden. Aufgrund der Kenntnis der Diagnose oder Diagnosen der das Medikament einnehmenden Person, der Indikation, erwarteter Nebenwirkungen aufgrund der Einnahme des Medikamentes und einer Kontraindikation des Medikamentes können die für die Beschreibung der zu erwarteten Wirkung des Medikamentes geeigneten Daten abgeleitet werden.

Ist beispielsweise das Medikament ein Betablocker, das die Person zum Senken ihres Blutdrucks einnimmt, da sie an Bluthochdruck leidet, umfassen die zur Beschreibung der tatsächlichen Wirkung des Medikamentes geeigneten Daten, d.h. des Betablockers, einen gemessenen Blutdruck und eine gemessene Herzfrequenz der Person, da bei einer Einnahme eines Betablockers Bradykardie (langsamer Herzschlag) als Nebenwirkung auftreten kann. Als weitere Nebenwirkung eines Betablockers kann orthostatische Dysregulation (Schwindelgefühl, insbesondere beim Aufstehen) auftreten. Folglich kann als allgemeines Wohlbefinden der Person insbesondere auftretender Schwindel erfasst werden.

Ein Vorteil der Erfindung ist, dass die das Medikament einnehmende Person zuverlässig überwacht und somit besser medizinisch geführt werden kann. Medikamentenwirkung, Nebenwirkungen oder Komplikationen können daher zuverlässig erfasst, erkannt und folglich geeignete Gegenmaßnahmen rechtzeitig ergriffen werden.

Die Fernüberwachung kann von verschiedenen Anbietern allein oder in Zusammenarbeit erbracht werden.

Ausführungsbeispiele sind exemplarisch in den schematischen Zeichnungen dargestellt. Es zeigen:
- Fig. 1: ein das erfindungsgemäße Verfahren und das erfindungsgemäße System veranschaulichende Szenario,
- Fig. 2: ein das erfindungsgemäße Verfahren veranschaulichendes Flussdiagramm,
- Fig. 3: eine Webseite und
- Fig. 4: ein das Anlegen einer elektronischen Überwachungsakte veranschaulichendes Diagramm.

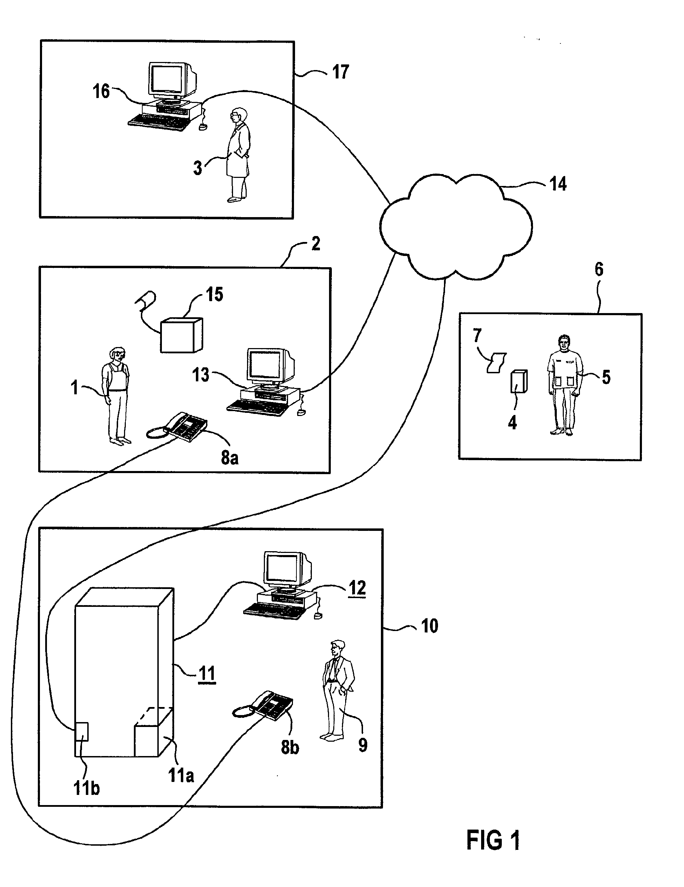

Die Fig. 1 zeigt einen Patienten 1, der sich im Falle des vorliegenden Ausführungsbeispieles in seiner Wohnung 2 aufhält und an Bluthochdruck leidet. Deshalb suchte er einen Arzt 3 in dessen Arztpraxis 17 auf. Der Arzt 3 untersuchte den Patienten 1 und verschrieb ihm ein den Blutdruck senkendes Medikament (Betablocker) 4, das der Patient 1 von einem Apotheker 5 in dessen Apotheke 6 abholte.

Im Falle des vorliegenden Ausführungsbeispieles umfasst ein Beipackzettel 7 des Betablockers 4 ein Angebot des Herstellers des Betablockers 4, die Wirkung des Betablockers 4 während dessen Einnahme fernüberwachen zu lassen. Um das Angebot der Fernüberwachung anzunehmen, wählt der Patient 1 mit einem in seiner Wohnung 2 befindlichen Telefon 8a eine auf dem Beipackzettel 7 angegebene Telefonnummer, worauf sich ein Angestellter 9 des Herstellers des Betablockers 4 mit einem in einem Geschäftsraum 10 des Herstellers befindlichen Telefons 8b meldet.

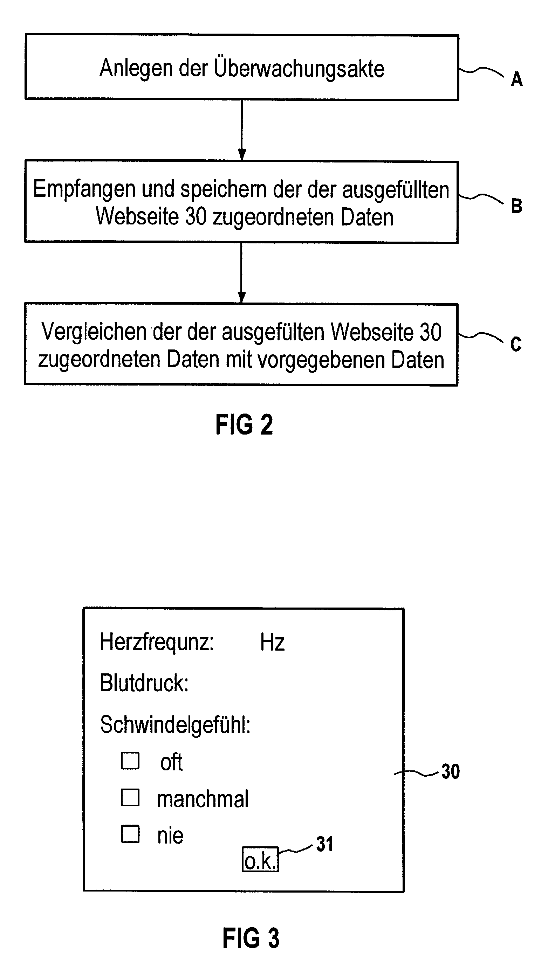

Nachdem im Falle des vorliegenden Ausführungsbeispiels der Angestellte von dem Patienten 1 erfuhr, dass der Patient 1 an Bluthochdruck leidet, den Betablocker 4 von dem Arzt 3 verschrieben bekommen hat und das Angebot der Fernüberwachung annehmen möchte, legt der Angestellte 9 mit einem in dem Geschäftsraum 10 befindlichen PC 12 eine dem Patienten 1 zugeordnete Überwachungsakte an. Die Überwachungsakte wird im Falle des vorliegenden Ausführungsbeispiels in einer Datenbank 11a einer mit dem PC 12 verbundenen Datenverarbeitungsanlage 11 gespeichert (Schritt A des in der Fig. 2 dargestellten Flussdiagramms).

Damit der Angestellte 9 ein geeignetes Fernüberwachungsprogramm für den Patienten 1 erstellen, d.h. geeignete Überwachungsparameter festlegen kann, sind im Falle des vorliegenden Ausführungsbeispiels in der Datenbank 11a Informationen über von dem Hersteller vertriebene Medikamente gespeichert. Diese Informationen umfassen im Falle des vorliegenden Ausführungsbeispiels u.a. Angaben über einen Grund der Einnahme, eine gewünschte Wirkung und eventuell auftretende Nebenwirkungen der von dem Hersteller vertrieben Medikamente.

Im Falle des vorliegenden Ausführungsbeispiels ermittelt der Angestellte 9 aufgrund der in der Datenbank 11a gespeicherten Informationen, dass bei einer Einnahme des Betablockers 4 Bradykardie (langsamer Herzschlag) und orthostatische Dysregulation (Schwindelgefühl, insbesondere beim Aufstehen) als unerwünschte Nebenwirkungen auftreten können. Die gewünschte Wirkung des Betablockers 4 ist eine Normalisierung des Blutdruckes. Folglich informiert der Angestellte 9 den Patienten 1, dass der Patient 1 einmal täglich seinen Blutdruck und seine Herzfrequenz messen und Angaben über den gemessenen Blutdruck und der gemessenen Herzfrequenz täglich an die Datenverarbeitungsanlage 11 übermitteln soll.

Damit der Patient 1 seinen Blutdruck messen kann, stellt ihm der Angestellte 9 während der Zeitdauer der Fernüberwachung ein Blutdruckmessgerät 15 zur Verfügung. Für eine zuverlässige und für den Patienten 1 bequeme Übermittlung des gemessenen Blutdrucks und der gemessenen Herzfrequenz richtet der Angestellte 9 im Falle des vorliegenden Ausführungsbeispiels eine dem Patienten 1 und in der Fig. 3 dargestellte Webseite 30, die in der Datenverarbeitungsanlage 11 gespeichert ist, ein. Die Webseite 30 kann der Patient 1 im Falle des vorliegenden Ausführungsbeispieles mit einem an das Internet 14 angeschlossenen und in der Wohnung 2 des Patienten 1 befindlichen PC 13 von der Datenverarbeitungsanlage 11 in allgemein bekannter Weise herunterladen. Die Datenverarbeitungsanlage 11 ist im Übrigen über einen geeigneten Internetanschluss 11b ebenfalls an das Internet 14 angeschlossen.

Im Falle des vorliegenden Ausführungsbeispiels misst also der Patient 1 täglich seinen Blutdruck und seine Herzfrequenz und trägt die gemessenen Werte in die Webseite 30, die er täglich mit seinem PC 13 von der Datenverarbeitungsanlage 11 herunter lädt, ein. Mittels der Webseite 30 gibt der Patient 1 ferner an, ob ihm oft, manchmal oder nie schwindlig ist. Die ausgefüllte Webseite 30 umfasst also Angaben bzw. Daten, die zur Beschreibung der Wirkung des Betablockers 4 geeignet sind.

Nachdem der Patient 1 die Webseite 30 ausgefüllt hat, übermittelt er die ausgefüllte Webseite 30 an die Datenverarbeitungsanlage 11, indem er das Feld 31 der Webseite 30 in allgemein bekannter Weise mit einer Rechnermaus des PCs 13 anklickt.

Auf der Datenverarbeitungsanlage 11 läuft des Weiteren ein geeignetes Rechnerprogramm, welches einen rechtzeitigen Eingang von zur Fernüberwachung von Kunden des Herstellers bestimmter Daten überwacht. Im Falle des vorliegenden Ausführungsbeispiels überwacht das auf der Datenverarbeitungsanlage 11 laufende Rechnerprogramm, ob der Patient 1 täglich eine ausgefüllte Webseite 30 an die Datenverarbeitungsanlage 11 übermittelt. Bei einem Ausbleiben einer ausgefüllten Webseite 30 generiert das Rechnerprogramm automatisch eine E-Mail, die eine Aufforderung zum Übermitteln der ausgefüllten Webseite 30 umfasst und automatisch an den PC 13 den Patienten 1 gesendet wird.

Nachdem die ausgefüllte Webseite 30 von der Datenverarbeitungsablage 11 empfangen und in der Datenbank 11a gespeichert wurde, überprüft das auf der Datenverarbeitungsanlage 11 laufende Rechnerprogramm, ob der Betablocker 4 wie gewünscht wirkt, also ob sich der Blutdruck des Patienten 1 normalisiert, und ob die unerwünschten Nebenwirkungen eintreten, also ob die Herzfrequenz des Patienten 1 zu niedrig oder ob dem Patienten 1 öfter schwindlig ist. Das Rechnerprogramm führt diese Überprüfung durch, indem es den Wert des gemessenen Blutdrucks mit einem Wert vergleicht, der einem normalen Blutdruck entspricht, den Wert der gemessenen Herzfrequenz mit einem Wert vergleicht, der einer niedrigen, aber noch normalen Herzfrequenz entspricht und überprüft, ob der Patient 1 angab, dass es ihm oft schwindlig ist (Schritte B und C des in der Fig. 2 dargestellten Flussdiagramms).

Ergibt im Falle des vorliegenden Ausführungsbeispiels die Überprüfung, dass die gewünschte Wirkung des Betablockers 4 nicht eintritt oder dass eine Nebenwirkung eintritt, generiert das auf der Datenverarbeitungsanlage 11 laufende Rechnerprogramm automatisch eine Nachricht. Diese Nachricht umfasst im Falle des vorliegenden Ausführungsbeispiels eine Angabe darüber, ob und welche Nebenwirkung auftrat bzw. dass die gewünschte Wirkung des Betablockers 4 nicht eintrat. Diese Nachricht wird im Falle des vorliegenden Ausführungsbeispiels in Form einer E-Mail von der Datenverarbeitungsanlage 11 an einen PC 16, der ebenfalls an das Internet 14 angeschlossen ist und sich in der Arztpraxis 17 des den Patienten 1 behandelnden Arztes 3 befindet, gesendet. Daher wird der Arzt 3 zuverlässig darüber informiert, ob der Betablocker 4 wie gewünscht wirkt oder ob unerwünschte Nebenwirkungen auftreten und kann somit nötigenfalls geeignet reagieren.

Nachdem die Fernüberwachung des Patienten 1 beendet ist, generiert ein weiteres, in der Datenverarbeitungsanlage 11 gespeichertes Rechnerprogramm automatisch eine der Fernüberwachung des Patienten 1 zugeordnete Rechnung, welche anschließend an eine Krankenkasse des Patienten 1 übermittelt wird.

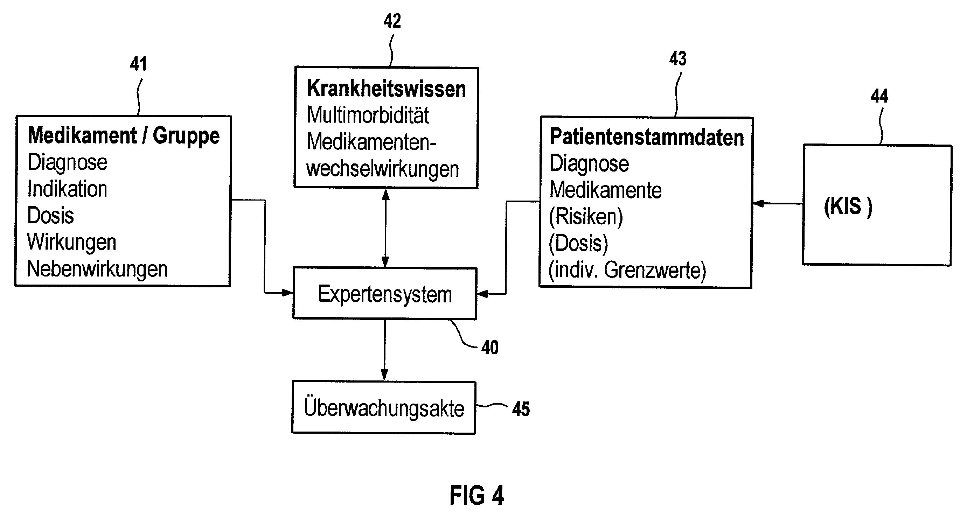

Im obenstehend beschriebenen Ausführungsbeispiel ermittelt der Angestellte 9 die zur Fernüberwachung des Patienten 1 geeigneten Überwachungsparameter mittels der in der Datenbank 11a der Datenverarbeitungsanlage 11 gespeicherten Informationen. Die Ermittlung der Überwachungsparameter kann aber auch mittels eines Expertensystems 40 erfolgen, wie es schematisch und exemplarisch in der Fig. 4 dargestellt ist.

Damit das in der Fig. 40 gezeigte Expertensystem 40 geeignete Überwachungsparameter ermitteln kann, kann es im Falle des vorliegenden Ausführungsbeispiels auf Daten, die in Listen 41, 42 und 43 hinterlegt sind, zugreifen. Die Listen 41, 42 und 43 sind z.B. in einer geeigneten Datenbank gespeichert.

Im Falle des vorliegenden Ausführungsbeispiels umfasst die Liste 42 Angaben über das verschriebene Medikament bzw. Medikamentengruppe. Diese Angaben umfassen Informationen über eine Diagnose, aufgrund derer das Medikament verschrienen wurde, eine Indikation, eine Dosis, gewünschte Wirkungen, unerwünschte Nebenwirkungen, usw.. Aus diesen Informationen lassen sich die erforderlichen Messparameter und Messfrequenzen ermitteln.

Die Liste 42 umfasst im Falle des vorliegenden Ausführungsbeispiels Angaben über ein Krankheitswissen, das z.B. Informationen über Medikamentenwechselwirkungen oder Multimorbität umfassen.

Die Liste 43 umfasst im Falle des vorliegenden Ausführungsbeispiels Patientenstammdaten, die dem zu überwachenden Patienten 1 zugeordnet sind und Informationen über eine Diagnose des Patienten 1, von dem Patienten 1 eingenommene Medikamente, eventuelle Risiken, Dosis oder individuelle Grenzwerte umfassen. Die Patientenstammdaten können im Übrigen, wie es in der Fig. 4 dargestellt ist, von einem Krankenhausinformationssystem (KIS) 44 bereit gestellt werden.

Aufgrund der in den Listen 41, 42 und 43 gespeicherten Daten kann das Expertensystem 40 die für die Überwachung des Patienten 1 notwendigen Daten generieren und somit eine geeignete Überwachungsakte 45 anlegen.

Wenn bei Multimorbität mehrere Medikamente verschrieben werden, kann es z.B. sein, dass derselbe Überwachungsparameter für zwei verschiedene Medikamente ermittelt wird. Dabei können unterschiedliche Messintervalle erforderlich sein, die beispielsweise von einer Halbwertzeit der Medikamente abhängen. Das Expertensystem 40 erkennt solche Situationen und kann gegebenenfalls geeignete Messzeiträume errechnen.

Das erfindungsgemäße Verfahren und das erfindungsgemäße System können bei der Fernüberwachung verschiedener Medikamente verwendet werden. Der Betablocker 4 ist nur als Beispiel genannt. Insbesondere können das erfindungsgemäße Verfahren oder das erfindungsgemäße System bei Medikamenten zur Behandlung chronischer Krankheiten (Hypertonie, Asthma, COLD, Diabetes, Herzinsuffizienz, Epilepsie, usw.), bei Medikamenten zur Behandlung akuter Zustände (z.B. Schmerzen oder Durchfall), bei einer Erfassung von Medikamentennebenwirkungen, im Rahmen einer Krebstherapie oder im Rahmen einer Schmerztherapie eingesetzt werden.

Für Studien, insbesondere für Medikamentenwirksamkeitsstudien, können während der Fernüberwachung Studienprotokolle hinterlegt sein. Aufgrund der Studienprotokolle kann festgestellt werden, welche Daten von der fernüberwachten Person zu welchen Zeitpunkten zu erfassen sind. Während der Medikamentenverträglichkeitsstudie kann dann insbesondere in der Datenverarbeitungsanlage 11 eine Webseite gespeichert sein, mittels derer sich an der Medikamentenverträglichkeitsstudie teilnehmende Personen über auftretende Nebenwirkungen von Medikamenten im Rahmen der Medikamentenverträglichkeitsstudie informieren können. In der Datenverarbeitungsanlage 11 kann aber auch eine weitere Webseite vorgesehen sein, auf der die an der Medikamentenverträglichkeitsstudie teilnehmenden Personen über Nebenwirkungen berichten können.

Die Ausführungsbeispiele sind im Übrigen nur exemplarisch zu verstehen.

## Patentansprüche

1. Verfahren zum Fernüberwachen einer Wirkung eines Medikamentes, aufweisend folgende Verfahrensschritte:
a) Anlegen einer elektronischen Überwachungsakte (45), die einer das Medikament (4) einnehmenden Person (1) zugeordnet ist,
b) Empfangen von für eine Beschreibung der tatsächlichen Wirkung des Medikamentes (4) geeigneten Daten,
c) Speichern der die Beschreibung der tatsächlichen Wirkung des Medikamentes (4) geeigneten Daten in der elektronischen Überwachungsakte (45) und
d) Vergleichen der die Beschreibung der tatsächlichen Wirkung des Medikamentes (4) geeigneten Daten mit für eine Beschreibung einer erwarteten Wirkung des Medikamentes (4) geeigneten Daten.

2. Verfahren nach Anspruch 1, bei dem den die Beschreibung der erwarteten Wirkung des Medikamentes (4) geeigneten Daten einer Diagnose, aufgrund derer die Person (1) das Medikament (4) einnimmt, einer Dosis des Medikamentes (4) und/oder einer zu erwartenden Nebenwirkung zugeordnet sind.

3. Verfahren nach Anspruch 1 oder 2, bei dem die erwartete Wirkung des Medikamentes (4) ein erwarteter medizinischer Zustand der das Medikament (4) einnehmenden Person (1) ist.

4. Verfahren nach einem der Ansprüche 1 bis 3, bei dem die Beschreibung der tatsächlichen Wirkung des Medikamentes (4) eine Beschreibung eines tatsächlichen medizinischen Zustands der das Medikament (4) einnehmenden Person (1) ist.

5. Verfahren nach Anspruch 4, bei dem der tatsächliche medizinische Zustand der Person (1) aufgrund des Blutdrucks, der Herzfrequenz, einem Brechreiz, eines Stuhlgangs, von Blutwerten und/oder eines allgemeinen Wohlbefindens der das Medikament (4) einnehmenden Person (1) ermittelt wird.

6. Verfahren nach Anspruch 4 oder 5, bei dem der tatsächliche medizinische Zustand der Person (1) mit wenigstens einem Messgerät (15) ermittelt wird.

7. Verfahren nach einem der Ansprüche 4 bis 6, bei dem die das Medikament (4) einnehmende Person (1) oder eine weitere, insbesondere im Gesundheitswesen tätige Person den tatsächlichen medizinischen Zustand ermittelt.

8. Verfahren nach einem der Ansprüche 1 bis 7, bei dem die die Beschreibung der tatsächlichen Wirkung des Medikamentes (4) geeigneten Daten über eine Datenübertragungseinrichtung (14) an eine die elektronische Überwachungsakte (45) umfassende Datenverarbeitungsanlage (11) übermittelt wird.

9. Verfahren nach einem der Ansprüche 1 bis 8, bei dem ein Alarmsignal ausgelöst wird, wenn die Wirkung des Medikamentes (4) von der erwarteten Wirkung um mehr als ein vorgegebenes Maß abweicht.

10. Verfahren nach einem der Ansprüche 1 bis 9, bei dem das Medikament (4) während einer Medikamentenwirksamkeitsstudie insbesondere während der Phase III und IV eingenommen wird.

11. Verfahren nach Anspruch 10, bei dem eine Webseite vorgesehen ist, mittels derer sich insbesondere das Medikament (4) einnehmende Personen während der Medikamentenverträglichkeitsstudie über auftretende Nebenwirkungen informieren können.

12. Verfahren nach Anspruch 10 oder 11, bei dem eine Webseite vorgesehen ist, auf der das Medikament einnehmende Personen während der Medikamentenverträglichkeitsstudie von aufgrund der Einnahme des Medikamentes (4) hervorgerufener Nebenwirkungen berichten können.

13. System zum Fernüberwachen einer Wirkung eines Medikamentes, das eine Person einnimmt, aufweisend
- eine Datenbank (11a), in der eine der Person (1) und dem Medikament (4) zugeordnete elektronische Überwachungsakte (45) und für eine Beschreibung einer erwarteten Wirkung des Medikamentes (4) geeignete Daten gespeichert sind,
- Mittel (11b) zum Empfangen von für die Beschreibung der tatsächlichen Wirkung des Medikamentes (4) geeigneten Daten und
- Mittel zum Vergleichen der für die Beschreibung der tatsächlichen Wirkung des Medikamentes (4) geeigneten Daten mit den für die Beschreibung der erwarteten Wirkung des Medikamentes (4) geeigneten Daten.

14. System nach Anspruch 13, bei dem für die Beschreibung der erwarteten Wirkung des Medikamentes (4) geeigneten Daten einer Diagnose, aufgrund deren die Person (1) das Medikament (4) einnimmt, einer Dosis des Medikamentes (4) und/oder einer zu erwartenden Nebenwirkung zugeordnet sind.

15. System nach Anspruch 13 oder 14, bei dem die erwartete Wirkung des Medikamentes (4) ein erwarteter medizinischer Zustand der das Medikament einnehmenden Person (1) ist.

16. System nach einem der Ansprüche 13 bis 15, bei dem die Beschreibung der tatsächlichen Wirkung des Medikamentes (4) geeigneten Daten eine Beschreibung eines tatsächlichen medizinischen Zustands der das Medikament (4) einnehmenden Person (1) ist.

17. System nach Anspruch 16, bei dem der tatsächliche medizinische Zustand aufgrund des Blutdrucks, der Herzfrequenz, einem Brechreiz, eines Stuhlgangs, von Blutwerten und/oder eines allgemeinen Wohlbefindens der das Medikament einnehmenden Person (1) beschrieben wird.

18. System nach Anspruch 16 oder 17, bei dem der tatsächliche medizinische Zustand mit wenigstens einem Messgerät (15) ermittelt wird.

19. System nach einem der Ansprüche 13 bis 16, bei dem die das Medikament (4) einnehmende Person (1) oder eine weitere, insbesondere im Gesundheitswesen tätige Person die tatsächliche Wirkung des Medikamentes (4) ermittelt.

20. System nach einem der Ansprüche 13 bis 19, bei dem die Mittel (11b) zum Empfangen von für die Beschreibung der tatsächlichen Wirkung des Medikamentes (4) geeigneten Daten derart ausgeführt sind, dass die für die Beschreibung der tatsächlichen Wirkung des Medikamentes (4) geeigneten Daten über eine Datenübertragungseinrichtung (14) übermittelbar sind.

21. System nach einem der Ansprüche 13 bis 20, das Mittel zum Auslösen eines Alarmsignals aufweist, wenn die Wirkung des Medikamentes (4) von der erwarteten Wirkung um mehr als ein vorgegebenes Maß abweicht.

22. System nach einem der Ansprüche 13 bis 21, das zur Überwachung der Wirkung des Medikamentes (4) während einer Medikamentenwirksamkeitsstudie insbesondere während der Phase III und IV geeignet ist.

23. Rechnerprogramm, das ein Verfahren nach einem der Ansprüche 1 bis 11 implementiert.

24. Rechnerprogramm, das ein System nach einem der Ansprüche 13 bis 22 implementiert.

25. Datenverarbeitungsanlage, auf dem ein Rechnerprogramm nach Anspruch 23 oder 24 gespeichert ist.
